# EUROPEAN PATENT SPECIFICATION

(11) **EP 3 547 858 B1**
(45) Date of publication and mention of the grant of the patent: **30.04.2025**
(21) Application number: 17817156.7
(22) Date of filing: 28.11.2017
(51) Int. Cl.: A24F 40/50

(54) **RECHARGEABLE LITHIUM-ION CAPACITOR FOR AN AEROSOL DELIVERY DEVICE**
WIEDERAUFLADBARER LITHIUM-IONEN-KONDENSATOR FÜR EINE AEROSOLABGABEVORRICHTUNG
CONDENSATEUR LITHIUM-ION RECHARGEABLE POUR UN DISPOSITIF DE DISTRIBUTION D'AÉROSOL

(30) Priority: 01.12.2016 US 201615366730
(43) Date of publication of application: 09.10.2019
(73) Proprietor: RAI Strategic Holdings, Inc., Winston-Salem, NC 27101 (US)
(72) Inventor: SUR, Rajesh, Winston-Salem, North Carolina 27106 (US); HUNT, Eric T., Pfafftown, North Carolina 27040 (US); SEARS, Stephen B., Siler City, North Carolina 27344 (US)
(74) Representative: Dainty, Katherine Louise
(86) International application number: PCT/IB2017/057464
(87) International publication number: WO 2018/100495

(56) References cited:
- EP-A1- 2 100 525
- WO-A1-2016/118005
- WO-A1-2017/070039
- WO-A2-2013/138384

## Description

### TECHNOLOGICAL FIELD

The present disclosure relates to aerosol delivery devices such as smoking articles, and more particularly to aerosol delivery devices that may utilize electrically generated heat for the production of aerosol (e.g., smoking articles commonly referred to as electronic cigarettes). The smoking articles may be configured to heat an aerosol precursor, which may incorporate materials that may be made or derived from, or otherwise incorporate tobacco, the precursor being capable of forming an inhalable substance for human consumption.

### BACKGROUND

Many devices have been proposed through the years as improvements upon, or alternatives to, smoking products that require combusting tobacco for use. Many of those devices purportedly have been designed to provide the sensations associated with cigarette, cigar, or pipe smoking, but without delivering considerable quantities of incomplete combustion and pyrolysis products that result from the burning of tobacco. To this end, there have been proposed numerous alternative smoking products, flavor generators, and medicinal inhalers that utilize electrical energy to vaporize or heat a volatile material, or attempt to provide the sensations of cigarette, cigar, or pipe smoking without burning tobacco to a significant degree.

However, it may be desirable to provide aerosol delivery devices with improved electronics such as may extend usability of the devices.

### BRIEF SUMMARY

The present disclosure relates to aerosol delivery devices, methods of forming such devices, and elements of such devices. The present disclosure thus includes, without limitation, the following example implementations.

Aspects of the invention are disclosed in independent apparatus claim 1, namely an aerosol delivery device comprising one housing enclosing a reservoir configured to retain an aerosol precursor composition; an atomizer; a power source connected to an electrical load that includes the atomizer, the power source comprising a lithium-ion capacitorconfigured to provide power to the electrical load, a DC-to-DC converter connected to the lithium-ion capacitor, between the lithium-ion capacitor and electrical load; a diode connected to, and between, the DC-to-DC converter and electrical load, wherein the lithium-ion capacitor, the DC-to-DC converter, the diode, and the electrical load are connected in series; and a microprocessor configured to operate in an active mode in which the microprocessor is configured to direct power from the power source to the atomizer and thereby control the atomizer to activate and vaporize components of the aerosol precursor composition.

Moreover, the following embodiments are disclosed:
The aerosol delivery device of above, wherein the LIC has a capacity in the range of 200 to 400 farads.

The aerosol delivery device of above, wherein the DC-to-DC converter is a switching regulator.

The aerosol delivery device of above, wherein the power source further comprises a snubber circuit connected in parallel with the LIC.

The aerosol delivery device of above, wherein the DC-to-DC converter has an input and output, and the diode has an anode and a cathode, and wherein the input and output of the DC-to-DC converter are connected to respectively the LIC and the anode of the diode, and the cathode of the diode is connected to the electrical load.

The aerosol delivery device of above, wherein the power source further comprises terminals connectable with a source of energy from which the LIC is chargeable.

The aerosol delivery device of above, wherein the aerosol precursor composition comprises at least glycerin and nicotine.

Further aspects of the invention are disclosed in independent apparatus claim 8, namely a control body coupled or coupleable with a cartridge that isequipped with an atomizer and contains an aerosol precursor composition, the control body being coupled or coupleable with the cartridge to form an aerosol delivery device in which the atomizer is configured to activate and vaporize components of the aerosol precursor composition, the control body comprising a power source connected to an electrical load that includes the atomizer when the control body is coupled with the cartridge, the power source comprising a lithium-ion capacitor configured to provide power to the electrical load, a DC-to-DC converter connected to the lithium-ion capacitor, between the lithium-ion capacitor and electrical load, and a diode connected to, and between, the DC-to-DC converter and electrical load, wherein the lithium-ion capacitor, the DC-to-DC converter, the diode, and the electrical load are connected in series; and a microprocessor configured to operate in an active mode in which the control body is coupled with the cartridge, the microprocessor in the active mode being configured to direct power from the power source to the atomizer and thereby control the atomizer to activate and vaporize components of the aerosol precursor composition.

The following respective embodiments are disclosed:
The control body of above, wherein the LIC has a capacity in the range of 200 to 400 farads.

The control body of above, wherein the DC-to-DC converter is a switching regulator.

The control body of above, wherein the power source further comprises a snubber circuit connected in parallel with the LIC.

The control body of above, wherein the DC-to-DC converter has an input and output, and the diode has an anode and a cathode, and wherein the input and output of the DC-to-DC converter are connected to respectively the LIC and the anode of the diode, and the cathode of the diode is connected to the electrical load.

The control body of above, wherein the power source further comprises terminals connectable with a source of energy from which the LIC is chargeable.

The control body of above, wherein the aerosol precursor composition comprises at least glycerin and nicotine.

These and other features, aspects, and advantages of the present disclosure will be apparent from a reading of the following detailed description together with the accompanying drawings, which are briefly described below. The present disclosure includes any combination of two, three, four or more features or elements set forth in this disclosure, regardless of whether such features or elements are expressly combined or otherwise recited in a specific example implementation described herein. This disclosure is intended to be read holistically such that any separable features or elements of the disclosure, in any of its aspects and example implementations, should be viewed as combinable, unless the context of the disclosure clearly dictates otherwise.

Other example implementations, aspects and advantages will become apparent from the following detailed description taken in conjunction with the accompanying drawings which illustrate, by way of example, the principles of some described example implementations.

### BRIEF DESCRIPTION OF THE DRAWING(S)

Having thus described the disclosure in the foregoing general terms, reference will now be made to the accompanying drawings, which are not necessarily drawn to scale, and wherein:
FIG. 1 illustrates a side view of an aerosol delivery device including a cartridge coupled to a control body, according to an example implementation of the present disclosure;
FIG. 2 is a partially cut-away view of the aerosol delivery device according to various example implementations;
FIG. 3 illustrates various elements of a control body and cartridge of the aerosol delivery device, according to various example implementations; and
FIG. 4 illustrates a power source for the aerosol delivery device that includes a lithium-ion capacitor (LIC), according to example implementations.

### DETAILED DESCRIPTION

The present disclosure will now be described more fully hereinafter with reference to example implementations thereof. These example implementations are described so that this disclosure will be thorough and complete, and will fully convey the scope of the disclosure to those skilled in the art. Indeed, the disclosure may be embodied in many different forms and should not be construed as limited to the implementations set forth herein; rather, these implementations are provided so that this disclosure will satisfy applicable legal requirements. As used in the specification and the appended claims, the singular forms "a," "an," "the" and the like include plural referents unless the context clearly dictates otherwise. Also, while reference may be made herein to quantitative measures, values, geometric relationships or the like, unless otherwise stated, any one or more if not all of these may be absolute or approximate to account for acceptable variations that may occur, such as those due to engineering tolerances or the like.

As described hereinafter, example implementations of the present disclosure relate to aerosol delivery devices. Aerosol delivery devices according to the present disclosure use electrical energy to heat a material (preferably without combusting the material to any significant degree) to form an inhalable substance; and components of such systems have the form of articles most preferably are sufficiently compact to be considered hand-held devices. That is, use of components of preferred aerosol delivery devices does not result in the production of smoke in the sense that aerosol results principally from byproducts of combustion or pyrolysis of tobacco, but rather, use of those preferred systems results in the production of vapors resulting from volatilization or vaporization of certain components incorporated therein. In some example implementations, components of aerosol delivery devices may be characterized as electronic cigarettes, and those electronic cigarettes most preferably incorporate tobacco and/or components derived from tobacco, and hence deliver tobacco derived components in aerosol form.

Aerosol generating pieces of certain preferred aerosol delivery devices may provide many of the sensations (e.g., inhalation and exhalation rituals, types of tastes or flavors, organoleptic effects, physical feel, use rituals, visual cues such as those provided by visible aerosol, and the like) of smoking a cigarette, cigar or pipe that is employed by lighting and burning tobacco (and hence inhaling tobacco smoke), without any substantial degree of combustion of any component thereof. For example, the user of an aerosol generating piece of the present disclosure can hold and use that piece much like a smoker employs a traditional type of smoking article, draw on one end of that piece for inhalation of aerosol produced by that piece, take or draw puffs at selected intervals of time, and the like.

While the systems are generally described herein in terms of implementations associated with aerosol delivery devices such as so-called "e-cigarettes," it should be understood that the mechanisms, components, features, and methods may be embodied in many different forms and associated with a variety of articles. For example, the description provided herein may be employed in conjunction with implementations of traditional smoking articles (e.g., cigarettes, cigars, pipes, etc.), heat-not-burn cigarettes, and related packaging for any of the products disclosed herein. Accordingly, it should be understood that the description of the mechanisms, components, features, and methods disclosed herein are discussed in terms of implementations relating to aerosol delivery devices by way of example only, and may be embodied and used in various other products and methods.

Aerosol delivery devices of the present disclosure also can be characterized as being vapor-producing articles or medicament delivery articles. Thus, such articles or devices can be adapted so as to provide one or more substances (e.g., flavors and/or pharmaceutical active ingredients) in an inhalable form or state. For example, inhalable substances can be substantially in the form of a vapor (i.e., a substance that is in the gas phase at a temperature lower than its critical point). Alternatively, inhalable substances can be in the form of an aerosol (i.e., a suspension of fine solid particles or liquid droplets in a gas). For purposes of simplicity, the term "aerosol" as used herein is meant to include vapors, gases and aerosols of a form or type suitable for human inhalation, whether or not visible, and whether or not of a form that might be considered to be smoke-like.

In use, aerosol delivery devices of the present disclosure may be subjected to many of the physical actions employed by an individual in using a traditional type of smoking article (e.g., a cigarette, cigar or pipe that is employed by lighting and inhaling tobacco). For example, the user of an aerosol delivery device of the present disclosure can hold that article much like a traditional type of smoking article, draw on one end of that article for inhalation of aerosol produced by that article, take puffs at selected intervals of time, etc.

Aerosol delivery devices of the present disclosure generally include a number of components provided within an outer body or shell, which may be referred to as a housing. The overall design of the outer body or shell can vary, and the format or configuration of the outer body that can define the overall size and shape of the aerosol delivery device can vary. Typically, an elongated body resembling the shape of a cigarette or cigar can be a formed from a single, unitary housing or the elongated housing can be formed of two or more separable bodies. For example, an aerosol delivery device can comprise an elongated shell or body that can be substantially tubular in shape and, as such, resemble the shape of a conventional cigarette or cigar. In one example, all of the components of the aerosol delivery device are contained within one housing. Alternatively, an aerosol delivery device can comprise two or more housings that are joined and are separable. For example, an aerosol delivery device can possess at one end a control body comprising a housing containing one or more reusable components (e.g., an accumulator such as a rechargeable battery and/or rechargeable supercapacitor, and various electronics for controlling the operation of that article), and at the other end and removably coupleable thereto, an outer body or shell containing a disposable portion (e.g., a disposable flavor-containing cartridge). More specific formats, configurations and arrangements of components within the single housing type of unit or within a multi-piece separable housing type of unit will be evident in light of the further disclosure provided herein. Additionally, various aerosol delivery device designs and component arrangements can be appreciated upon consideration of the commercially available electronic aerosol delivery devices.

Aerosol delivery devices of the present disclosure most preferably comprise some combination of a power source (i.e., an electrical power source), at least one control component (e.g., means for actuating, controlling, regulating and ceasing power for heat generation, such as by controlling electrical current flow the power source to other components of the article - e.g., a microprocessor, individually or as part of a microcontroller), a heater or heat generation member (e.g., an electrical resistance heating element or other component, which alone or in combination with one or more further elements may be commonly referred to as an "atomizer"), an aerosol precursor composition (e.g., commonly a liquid capable of yielding an aerosol upon application of sufficient heat, such as ingredients commonly referred to as "smoke juice," "e-liquid" and "e-juice"), and a mouthend region or tip for allowing draw upon the aerosol delivery device for aerosol inhalation (e.g., a defined airflow path through the article such that aerosol generated can be withdrawn therefrom upon draw).

Alignment of the components within the aerosol delivery device of the present disclosure can vary. In specific implementations, the aerosol precursor composition can be located near an end of the aerosol delivery device which may be configured to be positioned proximal to the mouth of a user so as to maximize aerosol delivery to the user. Other configurations, however, are not excluded. Generally, the heating element can be positioned sufficiently near the aerosol precursor composition so that heat from the heating element can volatilize the aerosol precursor (as well as one or more flavorants, medicaments, or the like that may likewise be provided for delivery to a user) and form an aerosol for delivery to the user. When the heating element heats the aerosol precursor composition, an aerosol is formed, released, or generated in a physical form suitable for inhalation by a consumer. It should be noted that the foregoing terms are meant to be interchangeable such that reference to release, releasing, releases, or released includes form or generate, forming or generating, forms or generates, and formed or generated. Specifically, an inhalable substance is released in the form of a vapor or aerosol or mixture thereof, wherein such terms are also interchangeably used herein except where otherwise specified.

As noted above, the aerosol delivery device may incorporate a battery or other electrical power source to provide current flow sufficient to provide various functionalities to the aerosol delivery device, such as powering of a heater, powering of control systems, powering of indicators, and the like. The power source can take on various implementations. Preferably, the power source is able to deliver sufficient power to rapidly heat the heating element to provide for aerosol formation and power the aerosol delivery device through use for a desired duration of time. The power source preferably is sized to fit conveniently within the aerosol delivery device so that the aerosol delivery device can be easily handled. Additionally, a preferred power source is of a sufficiently light weight to not detract from a desirable smoking experience.

More specific formats, configurations and arrangements of components within the aerosol delivery devices of the present disclosure will be evident in light of the further disclosure provided hereinafter. Additionally, the selection and arrangement of various aerosol delivery device components can be appreciated upon consideration of commercially-available electronic aerosol delivery devices.

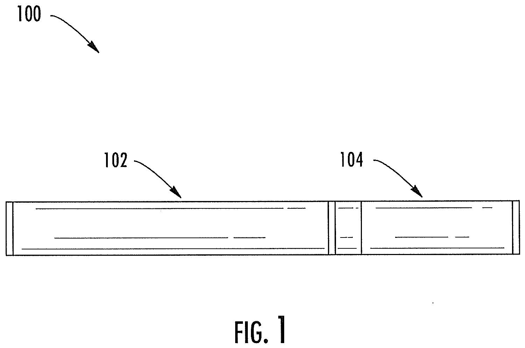

FIG. 1 illustrates a side view of an aerosol delivery device **100** including a control body **102** and a cartridge **104,** according to various example implementations of the present disclosure. In particular, FIG. 1 illustrates the control body and the cartridge coupled to one another. The control body and the cartridge may be detachably aligned in a functioning relationship. Various mechanisms may connect the cartridge to the control body to result in a threaded engagement, a press-fit engagement, an interference fit, a magnetic engagement or the like. The aerosol delivery device may be substantially rod-like, substantially tubular shaped, or substantially cylindrically shaped in some example implementations when the cartridge and the control body are in an assembled configuration. The aerosol delivery device may also be substantially rectangular, rhomboidal or triangular in cross-section, multifaceted shapes, or the like, some of which may lend itself to greater compatibility with a substantially flat or thin-film power source, such as a power source including a flat battery.

The cartridge and control body may include separate, respective housings or outer bodies, which may be formed of any of a number of different materials. The housing may be formed of any suitable, structurally-sound material. In some examples, the housing may be formed of a metal or alloy, such as stainless steel, aluminum or the like. Other suitable materials include various plastics (e.g., polycarbonate), metal-plating over plastic, ceramics and the like.

In some example implementations, one or both of the control body **102** or the cartridge 104 of the aerosol delivery device **100** may be referred to as being disposable or as being reusable. For example, the control body may have a replaceable battery or a rechargeable battery and thus may be combined with any type of recharging technology, including connection to a typical wall outlet, connection to a car charger (i.e., a cigarette lighter receptacle), connection to a computer, such as through a universal serial bus (USB) cable or connector, connection to a photovoltaic cell (sometimes referred to as a solar cell) or solar panel of solar cells, or connection to a RF-to-DC converter. Further, in some example implementations, the cartridge may comprise a single-use cartridge, as disclosed in U.S. Pat. No. 8,910,639 to Chang et al.

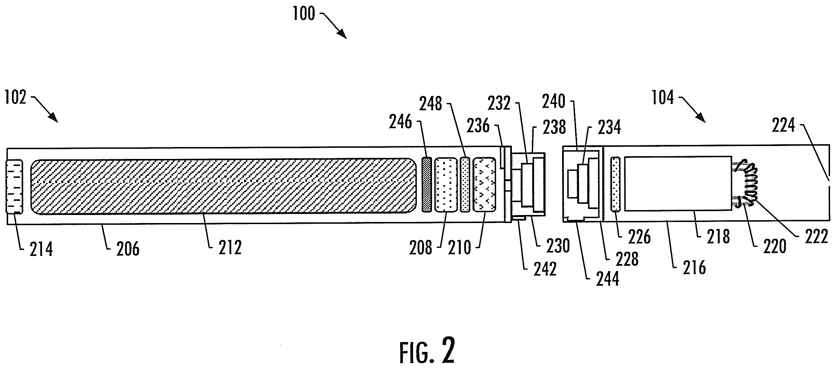

FIG. 2 more particularly illustrates the aerosol delivery device **100,** in accordance with some example implementations. As seen in the cut-away view illustrated therein, again, the aerosol delivery device can comprise a control body **102** and a cartridge **104** each of which include a number of respective components. The components illustrated in FIG. 2 are representative of the components that may be present in a control body and cartridge and are not intended to limit the scope of components that are encompassed by the present disclosure. As shown, for example, the control body can be formed of a control body shell 206 that can include a control component **208** (e.g., a microprocessor, individually or as part of a microcontroller), a flow sensor **210,** a power source **212** and one or more light-emitting diodes (LEDs) **214,** and such components can be variably aligned. The power source may include, for example, a battery (single-use or rechargeable), rechargeable supercapacitor, rechargeable solid-state battery (SSB), rechargeable lithium-ion battery (LiB) or the like, or some combination thereof.

The LED **214** may be one example of a suitable visual indicator with which the aerosol delivery device **100** may be equipped. Other indicators such as audio indicators (e.g., speakers), haptic indicators (e.g., vibration motors) or the like can be included in addition to or as an alternative to visual indicators such as the LED, quantum dot enabled LEDs.

The cartridge **104** can be formed of a cartridge shell **216** enclosing a reservoir **218** configured to retain the aerosol precursor composition, and including a heater **222** (sometimes referred to as a heating element). In various configurations, this structure may be referred to as a tank; and accordingly, the terms "cartridge," "tank" and the like may be used interchangeably to refer to a shell or other housing enclosing a reservoir for aerosol precursor composition, and including a heater.

As shown, in some examples, the reservoir **218** may be in fluid communication with a liquid transport element **220** adapted to wick or otherwise transport an aerosol precursor composition stored in the reservoir housing to the heater **222.** In some examples, a valve may be positioned between the reservoir and heater, and configured to control an amount of aerosol precursor composition passed or delivered from the reservoir to the heater.

Various examples of materials configured to produce heat when electrical current is applied therethrough may be employed to form the heater **222.** The heater in these examples may be a resistive heating element such as a wire coil, micro heater or the like. Example materials from which the heating element may be formed include Kanthal (FeCrAl), Nichrome, stainless steel, Molybdenum disilicide (MoSi₂), molybdenum silicide (MoSi), Molybdenum disilicide doped with Aluminum (Mo(Si,Al)₂), graphite and graphite-based materials (e.g., carbon-based foams and yarns) and ceramics (e.g., positive or negative temperature coefficient ceramics). Example implementations of heaters or heating members useful in aerosol delivery devices according to the present disclosure are further described below, and can be incorporated into devices such as those described herein.

An opening **224** may be present in the cartridge shell **216** (e.g., at the mouthend) to allow for egress of formed aerosol from the cartridge **104.**

The cartridge **104** also may include one or more electronic components **226,** which may include an integrated circuit, a memory component (e.g., EEPROM, flash memory), a sensor, or the like. The electronic components may be adapted to communicate with the control component **208** and/or with an external device by wired or wireless means. The electronic components may be positioned anywhere within the cartridge or a base **228** thereof.

Although the control component **208** and the flow sensor **210** are illustrated separately, it is understood that various electronic components including the control component and the flow sensor may be combined on an electronic printed circuit board (PCB) that supports and electrically connects the electronic components. Further, the PCB may be positioned horizontally relative the illustration of FIG. 1 in that the PCB can be lengthwise parallel to the central axis of the control body. In some examples, the air flow sensor may comprise its own PCB or other base element to which it can be attached. In some examples, a flexible PCB may be utilized. A flexible PCB may be configured into a variety of shapes, include substantially tubular shapes. In some examples, a flexible PCB may be combined with, layered onto, or form part or all of a heater substrate.

The control body **102** and the cartridge **104** may include components adapted to facilitate a fluid engagement therebetween. As illustrated in FIG. 2, the control body can include a coupler **230** having a cavity **232** therein. The base **228** of the cartridge can be adapted to engage the coupler and can include a projection **234** adapted to fit within the cavity. Such engagement can facilitate a stable connection between the control body and the cartridge as well as establish an electrical connection between the power source **212** and control component **208** in the control body and the heater **222** in the cartridge. Further, the control body shell **206** can include an air intake **236,** which may be a notch in the shell where it connects to the coupler that allows for passage of ambient air around the coupler and into the shell where it then passes through the cavity **232** of the coupler and into the cartridge through the projection **234.**

A coupler and a base useful according to the present disclosure are described in U.S. Pat. App. Pub. No. 2014/0261495 to Novak et al., which is incorporated herein by reference. For example, the coupler **230** as seen in FIG. 2 may define an outer periphery **238** configured to mate with an inner periphery **240** of the base **228.** In one example the inner periphery of the base may define a radius that is substantially equal to, or slightly greater than, a radius of the outer periphery of the coupler. Further, the coupler may define one or more protrusions **242** at the outer periphery configured to engage one or more recesses **244** defined at the inner periphery of the base. However, various other examples of structures, shapes and components may be employed to couple the base to the coupler. In some examples the connection between the base of the cartridge **104** and the coupler of the control body **102** may be substantially permanent, whereas in other examples the connection therebetween may be releasable such that, for example, the control body may be reused with one or more additional cartridges that may be disposable and/or refillable.

The reservoir **218** illustrated in FIG. 2 can be a container or can be a fibrous reservoir, as presently described. For example, the reservoir can comprise one or more layers of nonwoven fibers substantially formed into the shape of a tube encircling the interior of the cartridge shell **216,** in this example. An aerosol precursor composition can be retained in the reservoir. Liquid components, for example, can be sorptively retained by the reservoir. The reservoir can be in fluid connection with the liquid transport element **220.** The liquid transport element can transport the aerosol precursor composition stored in the reservoir via capillary action to the heater **222** that is in the form of a metal wire coil in this example. As such, the heater is in a heating arrangement with the liquid transport element. Example implementations of reservoirs and transport elements useful in aerosol delivery devices according to the present disclosure are further described below, and such reservoirs and/or transport elements can be incorporated into devices such as those described herein. In particular, specific combinations of heating members and transport elements as further described below may be incorporated into devices such as those described herein.

In use, when a user draws on the aerosol delivery device **100,** airflow is detected by the flow sensor **210,** and the heater **222** is activated to vaporize components of the aerosol precursor composition. Drawing upon the mouthend of the aerosol delivery device causes ambient air to enter the air intake **236** and pass through the cavity **232** in the coupler **230** and the central opening in the projection **234** of the base **228.** In the cartridge **104,** the drawn air combines with the formed vapor to form an aerosol. The aerosol is whisked, aspirated or otherwise drawn away from the heater and out the opening **224** in the mouthend of the aerosol delivery device.

In some examples, the aerosol delivery device **100** may include a number of additional software-controlled functions. For example, the aerosol delivery device may include a power-source protection circuit configured to detect power-source input, loads on the power-source terminals, and charging input. The power-source protection circuit may include short-circuit protection, under-voltage lock out and/or over-voltage charge protection, battery temperature compensation. The aerosol delivery device may also include components for ambient temperature measurement, and its control component **208** may be configured to control at least one functional element to inhibit power-source charging - particularly of any battery - if the ambient temperature is below a certain temperature (e.g., 0 °C) or above a certain temperature (e.g., 45 °C) prior to start of charging or during charging.

Power delivery from the power source **212** may vary over the course of each puff on the device **100** according to a power control mechanism. The device may include a "long puff" safety timer such that in the event that a user or component failure (e.g., flow sensor **210)** causes the device to attempt to puff continuously, the control component **208** may control at least one functional element to terminate the puff automatically after some period of time (e.g., four seconds). Further, the time between puffs on the device may be restricted to less than a period of time (e.g., 100 seconds). A watchdog safety timer may automatically reset the aerosol delivery device if its control component or software running on it becomes unstable and does not service the timer within an appropriate time interval (e.g., eight seconds). Further safety protection may be provided in the event of a defective or otherwise failed flow sensor **210,** such as by permanently disabling the aerosol delivery device in order to prevent inadvertent heating. A puffing limit switch may deactivate the device in the event of a pressure sensor fail causing the device to continuously activate without stopping after the four second maximum puff time.

The aerosol delivery device **100** may include a puff tracking algorithm configured for heater lockout once a defined number of puffs has been achieved for an attached cartridge (based on the number of available puffs calculated in light of the e-liquid charge in the cartridge). The aerosol delivery device may include a sleep, standby or low-power mode function whereby power delivery may be automatically cut off after a defined period of non-use. Further safety protection may be provided in that all charge/discharge cycles of the power source **212** may be monitored by the control component **208** over its lifetime. After the power source has attained the equivalent of a predetermined number (e.g., 200) of full discharge and full recharge cycles, it may be declared depleted, and the control component may control at least one functional element to prevent further charging of the power source.

The various components of an aerosol delivery device according to the present disclosure can be chosen from components described in the art and commercially available. Examples of batteries that can be used according to the disclosure are described in U.S. Pat. No. 9,484,155 to Peckerar et al.

The aerosol delivery device **100** can incorporate the sensor **210** or another sensor or detector for control of supply of electric power to the heater **222** when aerosol generation is desired (e.g., upon draw during use). As such, for example, there is provided a manner or method of turning off power to the heater when the aerosol delivery device is not be drawn upon during use, and for turning on power to actuate or trigger the generation of heat by the heater during draw.

The aerosol delivery device **100** most preferably incorporates the control component 208 or another control mechanism for controlling the amount of electric power to the heater **222** during draw.

The aerosol precursor composition, also referred to as a vapor precursor composition, may comprise a variety of components including, by way of example, a polyhydric alcohol (e.g., glycerin, propylene glycol or a mixture thereof), nicotine, tobacco, tobacco extract and/or flavorants.

Additional representative types of components that yield visual cues or indicators may be employed in the aerosol delivery device **100,** such as visual indicators and related components, audio indicators, haptic indicators and the like.

As indicated above, the control component **208** includes a number of electronic components, and in some examples may be formed of a PCB. The electronic components may include a microprocessor or processor core, and a memory. In some examples, the control component may include a microcontroller with integrated processor core and memory, and may further include one or more integrated input/output peripherals. In some examples, the control component may be coupled to a communication interface **246** to enable wireless communication with one or more networks, computing devices or other appropriately-enabled devices.

In accordance with some example implementations, the control component **208** may include or be coupled to a motion sensor **248** configured to detect a defined motion of the aerosol delivery device **100** that indicates a vulnerability of the aerosol delivery device. The motion sensor may be any of a number of sensors that may be configured to detect the defined motion, convert the defined motion to an electrical signal and output the electrical signal. Examples of suitable motion sensors include single or combinations of tilt sensors, single or multi-axis accelerometers, gyroscopes and the like, any one or more of which may be constructed using microelectromechanical systems-based (MEMS) techniques.

The motion sensor **248** may be configured to convert the defined motion to an electrical signal. The control component **208** or motion sensor may be configured to recognize the vulnerability and an operation associated with the vulnerability based on the electrical signal. In some examples, the defined motion detectable by the motion sensor may include vibration, shock or freefall. Consider in particular examples in which the motion sensor is an accelerometer. In these examples, vibration may be detectable by a periodic acceleration of at least a threshold amount. Additionally or alternatively, shock may be detectable by at least a threshold amount of acceleration for less than a threshold period of time, or freefall may be detectable by less than a threshold amount of acceleration for at least a threshold period of time.

The control component may then be configured to control at least one functional element of the aerosol delivery device **100** to perform the operation, which may be thereby performed in response to detection of the vulnerability. For example, the control component may be configured to shut off the power source **212,** which may be thereby shut off in response to detection of the vulnerability of the aerosol delivery device.

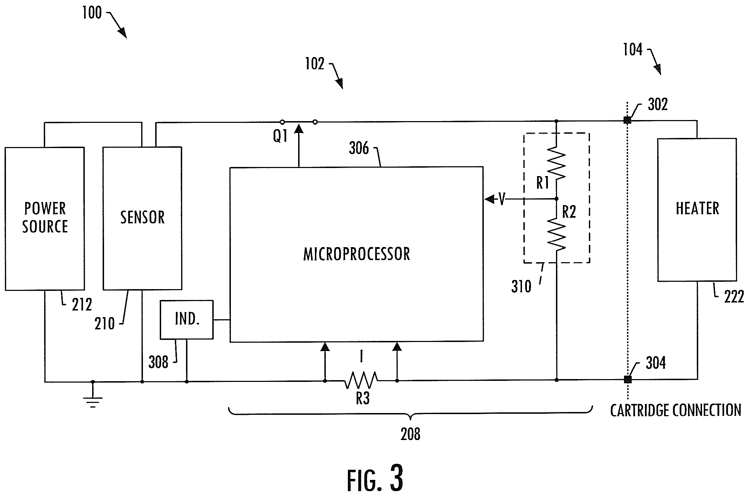

In accordance with some example implementations, the control component **208** may be configured to control one or more functional elements of the aerosol delivery device **100** in different states of the device. FIG. 3 illustrates the control body **102** coupled with the cartridge **104** in an active mode. As shown, the control body may include positive and negative terminals **302, 304** connectable with corresponding terminals of the heater **222** (heating element). The control component **208** may include a microprocessor **306** and a number of other electrical components, such as resistors, capacitors, switches and the like, which may be coupled with the power source **212** and heater to form an electrical circuit. In some examples, the heater may include a communication terminal for communicating data such as the puff count.

In accordance with example implementations of the present disclosure, the microprocessor **306** may be configured to measure the voltage at the positive terminal **302** and control power to the heater **222** based thereon. In some examples, the microprocessor may also control operation of at least one functional element of the aerosol delivery device **100** based on the voltage at the positive terminal. One example of a suitable functional element may be an indicator **308** such as a visual, audio or haptic indicator.

The microprocessor **306** may operate on the actual voltage at the positive terminal **302,** or an analog-to-digital converter (ADC) may be included to convert the actual voltage to a digital equivalent. In some examples, the ADC may be rated for a maximum voltage less than the maximum that may be present at the positive terminal. In these examples, the control component **208** may include a voltage divider **310** configured to reduce the voltage to the microprocessor. As shown, for example, the voltage divider may include resistors **R1** and **R2,** and may be connected to, and positioned between, the positive terminal and microprocessor, referenced to ground. The microprocessor may be configured to measure the voltage at the positive terminal from the voltage divider. In this regard, the voltage divider may include an output connected to the microprocessor and from which the microprocessor may be configured to measure the voltage at the positive terminal.

In examples in which the aerosol delivery device **100** has a housing formed of separable bodies, the aerosol delivery device, and more particularly the control component **102,** may be in the standby mode when the control component is uncoupled with the cartridge **104.** In examples of either a unitary or separable housing, the aerosol delivery device may be in the standby mode between puffs when the control component is coupled with the cartridge. Similarly, in examples of either a unitary or separable housing, when the user draws on the device and the flow sensor **210** detects airflow, the aerosol delivery device may be placed in the active mode during which power from the power source **212** may be directed through the sensor to power the heater **222** to activate and vaporize components of the aerosol precursor composition. In another example, power from the power source may more directly power the heater without going through the sensor (without the sensor being in-line), although the flow sensor may still detect airflow when the user draws on the device. As indicated above, power delivery from the power source may vary according to a power control mechanism; and in some examples, this power control mechanism may depend on a measured voltage at the positive terminal **302.**

In the active mode in which the control body **102** is coupled with the cartridge **104** (with a unitary or separable housing), the microprocessor **306** may be configured to direct power to the heater **222** to activate and vaporize components of the aerosol precursor composition. The voltage at the positive terminal **302** may correspond to a positive heater voltage. The microprocessor may be configured to measure the positive heater voltage, such as from the voltage divider **310,** and control the power directed to the heater based thereon.

In some more particular examples, the microprocessor **306** may be configured to direct power from the power source **212** (e.g., directly or through the flow sensor **210)** to turn the heater **222** on and commensurately initiate a heating time period. This may include, for example, a switch **Q1** between the power source (or in-line flow sensor) and the heater, which the microprocessor may operate in a closed state, as shown in FIG. 3. The microprocessor may then adjust the power directed to the heater based on the voltage at the positive terminal **302,** at a periodic rate until expiration of the heating time period.

In some examples, this adjustment of power directed to the heater **222** may include the microprocessor **306** being configured to determine a moving window of measurements of instantaneous actual power directed to the heater, with each measurement of the window of measurements being determined as a product of the positive heater voltage and a current through the heater. This current may be measured in a number of different manners, such as from a current-sense resistor **R3.** In some examples, the microprocessor may operate on the actual current through the heater, or the control component **208** or microprocessor may include an ADC configured to convert the actual current to a digital equivalent.

The microprocessor **306** may calculate a simple moving average power directed to the heater **222** based on the moving window of measurements of instantaneous actual power, and compare the simple moving average power to a selected power set point associated with the power source **212.** The microprocessor may then adjust the power directed to the heater so as to turn the heater off or on at the periodic rate at each instance in which the simple moving average power is respectively above or below the selected power set point.

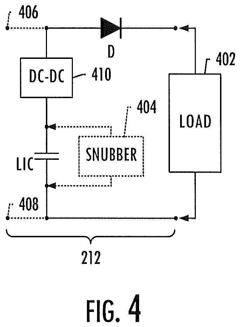

As indicated above, in some examples, the power source **212** includes a rechargeable supercapacitor. The supercapacitor may be any of a number of different types of supercapacitors, such as an electric double-layer capacitor (EDLC), a hybrid capacitor such as a lithium-ion capacitor (LIC), or the like. FIG. 4 more particularly illustrates the power source **212** including an LIC according to various example implementations of the present disclosure. As shown, the power source may be connected to an electrical load **402** that includes the heater **222** when the control body **102** is coupled with the cartridge **104.** More particularly, the electrical load may include the control component **208** (and its electrical components including the voltage divider **310)** and heater, which explained above, may be coupled with the power source to form an electrical circuit. This may additionally include, for example, the flow sensor **210,** indicator **308** and the like.

As shown, the power source **212** includes an LIC configured to provide power to the electrical load **402.** Hybrid capacitors such as the LIC generally have features of a battery such as high voltage and high energy density, while maintaining the traditional characteristics of a capacitor such as rapid charge (e.g., three seconds), high durability, safety and environmental friendliness. In some examples, the LIC has a capacity in the range of 200 to 400 farads, which gives it an energy density approximately 33% greater than that of a nominal LiB with the same or a similar form factor. A hybrid capacitor is also rechargeable, and has the ability to operate on its own for a longer period without the need of another source of energy from which the hybrid capacitor may be chargeable. The hybrid capacitor may also have a longer lifetime (e.g., 10 years) and cycle life as compared to other options, and is more environmentally friendly.

In some examples, a snubber circuit **404** may be connected in parallel with the LIC. In one example implementation, a LIC rated at 3.8 volts may include a parallel-connected snubber circuit to help maintain the LIC at least at 2.2 volts.

In some examples, the power source **212** may further include terminals **406, 408** connectable with a source of energy from which the LIC may be chargeable. The source of energy may be any of a number of different types, such as various rechargers configured to operate in a manner similar to a battery charger. In other examples, the source of energy may be or include a battery.

As also shown, in some examples, the power source **212** may further include other components such as a DC-to-DC converter **410** and/or a diode **D.** FIG. 4 illustrates the power source including both a DC-to-DC converter and diode, but it should be understood that the power source may include either without the other. In some examples, the DC-to-DC converter is a switching regulator, which may avoid too fast discharge of the LIC, and facilitate a uniform dissipation of current so that the LIC provides constant power to the electrical load **402.** The diode may facilitate current flow into the electrical load, and prevent current flow back, when the capacitor is discharged.

The DC-to-DC converter **410** may be connected to the LIC, between the LIC and electrical load **402.** As shown, in some more particular examples, the diode **D** may be connected to, and between, the DC-to-DC converter and electrical load. And in at least some of these examples, an input and output of the DC-to-DC converter may be connected to respectively the LIC and anode of the diode, and the cathode of the diode may be connected to the electrical load.

The foregoing description of use of the article(s) can be applied to the various example implementations described herein through minor modifications, which can be apparent to the person of skill in the art in light of the further disclosure provided herein. The above description of use, however, is not intended to limit the use of the article but is provided to comply with all necessary requirements of disclosure of the present disclosure. Any of the elements shown in the article(s) illustrated in FIGS. 1-4 or as otherwise described above may be included in an aerosol delivery device according to the present disclosure.

Many modifications and other implementations of the disclosure set forth herein will come to mind to one skilled in the art to which this disclosure pertains having the benefit of the teachings presented in the foregoing descriptions and the associated drawings. Therefore, it is to be understood that the disclosure is not to be limited to the specific implementations disclosed, and that modifications and other implementations are intended to be included within the scope of the appended claims. Moreover, although the foregoing descriptions and the associated drawings describe example implementations in the context of certain example combinations of elements and/or functions, it should be appreciated that different combinations of elements and/or functions may be provided by alternative implementations without departing from the scope of the appended claims.

## Claims

1. An aerosol delivery device (100) comprising:
at least one housing (216) enclosing a reservoir (218) configured to retain an aerosol precursor composition;
an atomizer (222);
a power source (212) connected to an electrical load that includes the atomizer (222), the power source (212) comprising a lithium-ion capacitor configured to provide power to the electrical load, a DC-to-DC converter (410) connected to the lithium-ion capacitor, between the lithium-ion capacitor and electrical load;
a diode (D) connected to, and between, the DC-to-DC converter (410) and electrical load, wherein the lithium-ion capacitor, the DC-to-DC converter (410), the diode (D), and the electrical load are connected in series; and
a microprocessor (306) configured to operate in an active mode in which the microprocessor (306) is configured to direct power from the power source (212) to the atomizer (222) and thereby control the atomizer (222) to activate and vaporize components of the aerosol precursor composition.

2. The aerosol delivery device (100) of Claim 1, wherein the lithium-ion capacitor has a capacity in the range of 200 to 400 farads.

3. The aerosol delivery device (100) of Claim 1 or 2, wherein the DC-to-DC converter (410) is a switching regulator.

4. The aerosol delivery device (100) of any one of Claims 1 to 3,
wherein the power source (212) further comprises a snubber circuit (404) connected in parallel with the lithium-ion capacitor.

5. The aerosol delivery device (100) of any one of Claims 1 to 4,
wherein the DC-to-DC converter (410) has an input and output, and the diode (D) has an anode and a cathode, and
wherein the input and output of the DC-to-DC converter (410) are connected to respectively the lithium-ion capacitor and the anode of the diode, and the cathode of the diode is connected to the electrical load.

6. The aerosol delivery device (100) of any one of Claims 1 to 5,
wherein the power source (212) further comprises terminals (406, 408) connectable with a source of energy from which the lithium-ion capacitor is chargeable.

7. The aerosol delivery device (100) of any one of Claims 1 to 6,
wherein the aerosol precursor composition comprises at least glycerin and nicotine.

8. A control body (102) coupled or coupleable with a cartridge (104) that is equipped with an atomizer (222) and contains an aerosol precursor composition, the control body (102) being coupled or coupleable with the cartridge (104) to form an aerosol delivery device (100) in which the atomizer (222) is configured to activate and vaporize components of the aerosol precursor composition, the control body (102) comprising:
a power source (212) connected to an electrical load that includes the atomizer (222) when the control body (102) is coupled with the cartridge (104), the power source (212) comprising a lithium-ion capacitor configured to provide power to the electrical load, a DC-to-DC converter (410) connected to the lithium-ion capacitor, between the lithium-ion capacitor and electrical load, and
a diode (D) connected to, and between, the DC-to-DC converter (410) and electrical load, wherein the lithium-ion capacitor, the DC-to-DC converter (410), the diode (D), and the electrical load are connected in series; and
a microprocessor (306) configured to operate in an active mode in which the control body (102) is coupled with the cartridge (104), the microprocessor (306) in the active mode being configured to direct power from the power source (212) to the atomizer (222) and thereby control the atomizer (222) to activate and vaporize components of the aerosol precursor composition.

9. The control body (102) of Claim 8, wherein the lithium-ion capacitor has a capacity in the range of 200 to 400 farads.

10. The control body (102) of Claim 8 or 9, wherein the DC-to-DC converter (410) is a switching regulator.

11. The control body (102) of any one of Claims 8 to 10, wherein the power source (212) further comprises a snubber circuit (404) connected in parallel with the lithium-ion capacitor.

12. The control body (102) of any one of Claims 8 to 11, wherein the DC-to-DC converter (410) has an input and output, and the diode (D) has an anode and a cathode, and
wherein the input and output of the DC-to-DC converter (410) are connected to respectively the lithium-ion capacitor and the anode of the diode (D), and the cathode of the diode (D) is connected to the electrical load.

13. The control body (102) of any one of Claims 8 to 12, wherein the power source (212) further comprises terminals (406, 408) connectable with a source of energy from which the lithium-ion capacitor is chargeable.

14. The control body (102) of any one of Claims 8 to 13, wherein the aerosol precursor composition comprises at least glycerin and nicotine.

## Patentansprüche

1. Aerosolabgabevorrichtung (100), umfassend:
mindestens ein Gehäuse (216), das einen Behälter (218) umschließt, der dazu konfiguriert ist, eine Aerosolvorläuferzusammensetzung vorzuhalten;
einen Zerstäuber (222);
eine Stromquelle (212), die an eine elektrische Last angeschlossen ist, die den Zerstäuber (222) beinhaltet, wobei die Stromquelle (212) einen Lithium-Ionen-Kondensator, der dazu konfiguriert ist, die elektrische Last mit Strom zu versorgen, einen Gleichspannungswandler (410), der an den Lithium-Ionen-Kondensator angeschlossen ist, zwischen dem Lithium-Ionen-Kondensator und der elektrischen Last umfasst;
eine Diode (D), die an den und zwischen dem Gleichspannungswandler (410) und der elektrischen Last angeschlossen ist, wobei der Lithium-Ionen-Kondensator, der Gleichspannungswandler (410), die Diode (D) und die elektrische Last in Reihe angeschlossen sind; und
einen Mikroprozessor (306), der dazu konfiguriert ist, in einem aktiven Modus zu operieren, in dem der Mikroprozessor (306) dazu konfiguriert ist, Strom von der Stromquelle (212) zu dem Zerstäuber (222) zu leiten und dadurch den Zerstäuber (222) zu steuern, um Komponenten der Aerosolvorläuferzusammensetzung zu aktivieren und zu verdampfen.

2. Aerosolabgabevorrichtung (100) nach Anspruch 1, wobei der Lithium-Ionen-Kondensator eine Kapazität im Bereich von 200 bis 400 Farad aufweist.

3. Aerosolabgabevorrichtung (100) nach Anspruch 1 oder 2, wobei der Gleichspannungswandler (410) ein Schaltregler ist.

4. Aerosolabgabevorrichtung (100) nach einem der Ansprüche 1 bis 3, wobei die Stromquelle (212) ferner eine Dämpferschaltung (404) umfasst, die mit dem Lithium-Ionen-Kondensator parallel angeschlossen ist.

5. Aerosolabgabevorrichtung (100) nach einem der Ansprüche 1 bis 4, wobei der Gleichspannungswandler (410) einen Eingang und einen Ausgang aufweist und die Diode (D) eine Anode und eine Kathode aufweist, und
wobei der Eingang und Ausgang des Gleichspannungswandlers (410) jeweils an den Lithium-Ionen-Kondensator und die Anode der Diode angeschlossen sind und die Kathode der Diode an die elektrische Last angeschlossen ist.

6. Aerosolabgabevorrichtung (100) nach einem der Ansprüche 1 bis 5, wobei die Stromquelle (212) ferner Anschlüsse (406, 408) umfasst, die mit einer Energiequelle verbindbar sind, aus der der Lithium-Ionen-Kondensator aufladbar ist.

7. Aerosolabgabevorrichtung (100) nach einem der Ansprüche 1 bis 6, wobei die Aerosolvorläuferzusammensetzung mindestens Glycerin und Nikotin umfasst.

8. Steuerkörper (102), der mit einer Patrone (104) gekoppelt oder koppelbar ist, die mit einem Zerstäuber (222) ausgerüstet ist und eine Aerosolvorläuferzusammensetzung enthält, wobei der Steuerkörper (102) mit der Patrone (104) gekoppelt oder koppelbar ist, um eine Aerosolabgabevorrichtung (100) zu bilden, in der der Zerstäuber (222) dazu konfiguriert ist, Komponenten der Aerosolvorläuferzusammensetzung zu aktivieren und zu verdampfen, wobei der Steuerkörper (102) umfasst:
eine Stromquelle (212), die an eine elektrische Last angeschlossen ist, die den Zerstäuber (222) beinhaltet, wenn der Steuerkörper (102) mit der Patrone (104) gekoppelt ist, wobei die Stromquelle (212) einen Lithium-Ionen-Kondensator, der dazu konfiguriert ist, die elektrische Last mit Strom zu versorgen, einen Gleichspannungswandler (410), der an den Lithium-Ionen-Kondensator angeschlossen ist, zwischen dem Lithium-Ionen-Kondensator und der elektrischen Last umfasst, und
eine Diode (D), die an den und zwischen dem Gleichspannungswandler (410) und der elektrischen Last angeschlossen ist, wobei der Lithium-Ionen-Kondensator, der Gleichspannungswandler (410), die Diode (D) und die elektrische Last in Reihe angeschlossen sind; und
einen Mikroprozessor (306), der dazu konfiguriert ist, in einem aktiven Modus zu operieren, in dem der Steuerkörper (102) mit der Patrone (104) gekoppelt ist, wobei der Mikroprozessor (306) in dem aktiven Modus dazu konfiguriert ist, Strom von der Stromquelle (212) zu dem Zerstäuber (222) zu leiten und dadurch den Zerstäuber (222) zu steuern, um Komponenten der Aerosolvorläuferzusammensetzung zu aktivieren und zu verdampfen.

9. Steuerkörper (102) nach Anspruch 8, wobei der Lithium-Ionen-Kondensator eine Kapazität im Bereich von 200 bis 400 Farad aufweist.

10. Steuerkörper (102) nach Anspruch 8 oder 9, wobei der Gleichspannungswandler (410) ein Schaltregler ist.

11. Steuerkörper (102) nach einem der Ansprüche 8 bis 10, wobei die Stromquelle (212) ferner eine Dämpferschaltung (404) umfasst, die mit dem Lithium-Ionen-Kondensator parallel angeschlossen ist.

12. Steuerkörper (102) nach einem der Ansprüche 8 bis 11, wobei der Gleichspannungswandler (410) einen Eingang und einen Ausgang aufweist und die Diode (D) eine Anode und eine Kathode aufweist, und
wobei der Eingang und Ausgang des Gleichspannungswandlers (410) jeweils an den Lithium-Ionen-Kondensator und die Anode der Diode (D) angeschlossen sind und die Kathode der Diode (D) an die elektrische Last angeschlossen ist.

13. Steuerkörper (102) nach einem der Ansprüche 8 bis 12, wobei die Stromquelle (212) ferner Anschlüsse (406, 408) umfasst, die mit einer Energiequelle verbindbar sind, aus der der Lithium-Ionen-Kondensator aufladbar ist.

14. Steuerkörper (102) nach einem der Ansprüche 8 bis 13, wobei die Aerosolvorläuferzusammensetzung mindestens Glycerin und Nikotin umfasst.

## Revendications

1. Dispositif de distribution d'aérosol (100) comprenant :
au moins un boîtier (216) renfermant un réservoir (218) configuré pour conserver une composition de précurseur d'aérosol ;
un atomiseur (222) ;
une source d'alimentation (212) connectée à une charge électrique qui comprend l'atomiseur (222), la source d'alimentation (212) comprenant un condensateur lithium-ion configuré pour fournir l'alimentation à la charge électrique, un convertisseur CC-CC (410) connecté au condensateur lithium-ion, entre le condensateur lithium-ion et la charge électrique ;
une diode (D) connectée au convertisseur CC-CC (410) et à la charge électrique, et entre ceux-ci, dans lequel le condensateur lithium-ion, le convertisseur CC-CC (410), la diode (D) et la charge électrique sont connectés en série ; et
un microprocesseur (306) configuré pour fonctionner dans un mode actif dans lequel le microprocesseur (306) est configuré pour diriger l'alimentation provenant de la source d'alimentation (212) vers l'atomiseur (222) et ainsi commander à l'atomiseur (222) d'activer et de vaporiser les composants de la composition de précurseur d'aérosol.

2. Dispositif de distribution d'aérosol (100) selon la revendication 1, dans lequel le condensateur lithium-ion comporte une capacité comprise dans la plage de 200 à 400 farads.

3. Dispositif de distribution d'aérosol (100) selon la revendication 1 ou 2, dans lequel le convertisseur CC-CC (410) est un régulateur à commutation.

4. Dispositif de distribution d'aérosol (100) selon l'une quelconque des revendications 1 à 3, dans lequel la source d'alimentation (212) comprend en outre un circuit d'amortissement (404) connecté en parallèle avec le condensateur lithium-ion.

5. Dispositif de distribution d'aérosol (100) selon l'une quelconque des revendications 1 à 4, dans lequel le convertisseur CC-CC (410) comporte une entrée et une sortie, et la diode (D) comporte une anode et une cathode, et
dans lequel l'entrée et la sortie du convertisseur CC-CC (410) sont connectées respectivement au condensateur lithium-ion et à l'anode de la diode, et la cathode de la diode est connectée à la charge électrique.

6. Dispositif de distribution d'aérosol (100) selon l'une quelconque des revendications 1 à 5, dans lequel la source d'alimentation (212) comprend en outre des bornes (406, 408) pouvant être connectées à une source d'énergie à partir de laquelle le condensateur lithium-ion peut être chargé.

7. Dispositif de distribution d'aérosol (100) selon l'une quelconque des revendications 1 à 6, dans lequel la composition de précurseur d'aérosol comprend au moins de la glycérine et de la nicotine.

8. Corps de commande (102) couplé ou pouvant être couplé à une cartouche (104) qui est équipée d'un atomiseur (222) et contient une composition de précurseur d'aérosol, le corps de commande (102) étant couplé ou pouvant être couplé à la cartouche (104) pour former un dispositif de distribution d'aérosol (100) dans lequel l'atomiseur (222) est configuré pour activer et vaporiser les composants de la composition de précurseur d'aérosol, le corps de commande (102) comprenant :
une source d'alimentation (212) connectée à une charge électrique qui comprend l'atomiseur (222) lorsque le corps de commande (102) est couplé à la cartouche (104), la source d'alimentation (212) comprenant un condensateur lithium-ion configuré pour fournir l'alimentation à la charge électrique, un convertisseur CC-CC (410) connecté au condensateur lithium-ion, entre le condensateur lithium-ion et la charge électrique, et
une diode (D) connectée au convertisseur CC-CC (410) et à la charge électrique, et entre ceux-ci, dans lequel le condensateur lithium-ion, le convertisseur CC-CC (410), la diode (D) et la charge électrique sont connectés en série ; et
un microprocesseur (306) configuré pour fonctionner dans un mode actif dans lequel le corps de commande (102) est couplé à la cartouche (104), le microprocesseur (306) dans le mode actif étant configuré pour diriger l'alimentation provenant de la source d'alimentation (212) vers l'atomiseur (222) et ainsi commander à l'atomiseur (222) d'activer et de vaporiser les composants de la composition de précurseur d'aérosol.

9. Corps de commande (102) selon la revendication 8, dans lequel le condensateur lithium-ion comporte une capacité comprise dans la plage de 200 à 400 farads.

10. Corps de commande (102) selon la revendication 8 ou 9, dans lequel le convertisseur CC-CC (410) est un régulateur à commutation.

11. Corps de commande (102) selon l'une quelconque des revendications 8 à 10, dans lequel la source d'alimentation (212) comprend en outre un circuit d'amortissement (404) connecté en parallèle avec le condensateur lithium-ion.

12. Corps de commande (102) selon l'une quelconque des revendications 8 à 11, dans lequel le convertisseur CC-CC (410) comporte une entrée et une sortie, et la diode (D) comporte une anode et une cathode, et
dans lequel l'entrée et la sortie du convertisseur CC-CC (410) sont connectées respectivement au condensateur lithium-ion et à l'anode de la diode (D), et la cathode de la diode (D) est connectée à la charge électrique.

13. Corps de commande (102) selon l'une quelconque des revendications 8 à 12, dans lequel la source d'alimentation (212) comprend en outre des bornes (406, 408) pouvant être connectées à une source d'énergie à partir de laquelle le condensateur lithium-ion peut être chargé.

14. Corps de commande (102) selon l'une quelconque des revendications 8 à 13, dans lequel la composition de précurseur d'aérosol comprend au moins de la glycérine et de la nicotine.
